# EUROPÄISCHE PATENTSCHRIFT

(11) **EP 1 491 191 B1**
(45) Veröffentlichungstag und Bekanntmachung des Hinweises auf die Patenterteilung: **26.09.2007**
(21) Anmeldenummer: 03014143.6
(22) Anmeldetag: 24.06.2003
(51) Int. Cl.: A61K 31/35, A23L 1/30

(54) **Extrakte von Litchi sinensis enthaltend oligomere Proanthocyanidine**
Extracts from Lychee (Litchi sinensis) containing oligomeric proanthocyanidines
Extrait de litchi contenant de proanthocyanidines oligomeriques

(43) Veröffentlichungstag der Anmeldung: 29.12.2004
(73) Patentinhaber: Cognis IP Management GmbH, 40589 Düsseldorf (DE)
(72) Erfinder: Rull Prous, Santiago, 08034 Barcelona (ES); Alaoui Ismaili, Smail, 08290 Cerdanyola del Vallès (ES); Fabry, Bernd, Dr., 41352 Korschenbroich (DE)

(56) Entgegenhaltungen:
- EP-A- 0 659 402
- EP-A- 0 713 706
- EP-A- 1 247 527
- US-A- 4 863 956
- US-A- 5 650 432
- DATABASE WPI Section Ch, Week 199935 Derwent Publications Ltd., London, GB; Class D16, AN 1999-405815 XP002259113 & CN 1 215 082 A (UNIV POLYTECHNIC COLLEGE HUANAN TROPICAL), 28. April 1999 (1999-04-28)
- DATABASE WPI Section Ch, Week 199739 Derwent Publications Ltd., London, GB; Class A97, AN 1997-416119 XP002259114 "Litchi taste instant drink" & CN 1 113 718 A (XIA M), 27. Dezember 1995 (1995-12-27)
- SARNI-MANCHADO P; LE ROUX E: "Phenolic composition of Litchi Fruit Pericap" AGRIC. FOOD CHEM., Bd. 48, 2000, Seiten 5995-6002, XP002259112

## Beschreibung

### Gebiet der Erfindung

Die Erfindung befindet sich auf dem Gebiet der Nahrungsmittelergänzungsstoffe und betrifft Verfahren zur Herstellung spezieller botanischer Extrakte mit einem hohen Gehalt an speziellen Wirkstoffen.

### Stand der Technik

Extrakte der Schalen von Pflanzen der Gattung *Sapindaceae,* speziell der Spezies *Litchi sinensis (Sonn.)* sind bekannt für ihren hohen Gehalt an Flavonderivaten, insbesondere an Hydrier- oder O-xidationsprodukten des 2-phenyl-4H-1-benzopyrans oder deren Derivaten, wie z.B. Flavanen, Flavan-3-olen (Catechinen, Catechinoligomeren), Flavan-3,4-diolen (Leucoanthocyaniden), Flavonen, Flavonolen and Flavononen. Den Hauptanteil der Extrakte stellen jedoch kondensierte Tannine, sogenannte "oligomere Procyanodole" (OPC) dar. Es handelt sich dabei um Oligomere mit 2 bis 8 Monomeren vom Catechin- oder Epicatechintyp, wie z.B. Procyanidine, Proanthocyanidine, Procyanidoel, Oligoprocyanidine, Leucoanthocyanidine, Leucodelphinine, Leucocyanine sowie Anthocyanogene. OPC, insbesondere das besonders wirksame Proanthocyanidin A2 (OPC A2), zeigen Eigenschaften, die denen des Vitamins P ähneln, insbesondere die Inhibierung Matrixmetalloproteasen (MMP), MMP haben aber die Eigenschaft, die dermalen Makromoleküle des Bindegewebes, wie Proteoglycan, Collagen und Elastin anzugreifen, die Peptidbindungen zu lösen und damit ursächlich zur Hautalterung beizutragen. Auch bei inflammatorischen Prozessen in der Haut werden von den Makrophagen und von polymorphonuclearen neutrophilen Granulocyten Proteasen wie z.B. die Serin-Protease Elastase oder Matrix-Metallo-Proteasen (MMP) wie Collagenase und eine weitere, zu den MMP gehörende Elastin abbauende Elastase ausgeschüttet.

In diesem Zusammenhang sei die europäische Patentanmeldung EP 0978274 A1 (Kibun) erwähnt, aus der kosmetische Zubereitungen für die topische Anwendung bekannt sind, die Emulgatoren vom Typ der Sphingoglycolipide zusammen mit Litchi-Extrakten enthalten, wobei letztere zur Depigmentierung der Haut dienen. Ferner werden in der Europäischen Patentanmeldung EP 0965328 A1 (Kao) kosmetische Mittel offenbart, die Litchi-Extrakte zusammen mit speziellen Phosphorsäureestem enthalten. Keine der beiden Schriften enthält einen Hinweis, auf welche Weise Litchi-Extrakte hergestellt werden können oder auf deren orale Anwendung im Bereich der Nahrungsmittelergänzungsstoffe.

Die Patentschrift US 5 650 432 A offenbart ein Verfahren zur Gewinnung von Proanthocyanidin Oligo- und Polymeren aus Pflanzenmaterial.

Die Herstellung von Lichi-Extrakten erweist sich in der Praxis als schwierig. Mit üblichen Techniken der Extraktion mit unterschiedlich polaren Lösemitteln werden nur Extrakte mit Gehalten bis zu 15 Gew.-% an OPC A2 erhalten, zu wenig, um die Extraktion wirtschaftlich durchzuführen und die Produkte unter ökonomisch vertretbaren Bedingungen zu vermarkten. Die Aufgabe der vorliegenden Erfindung hat somit darin bestanden, Extrakte von *Litchi sinensis* sowie ein Verfahren zu deren Herstellung zur Verfügung zu stellen, welche - bezogen auf die Aktivsubstanz - wenigstens 15, vorzugsweise wenigstens 20 und insbesondere 20 bis 25 Gew.-% an oligomeren Proanthocyanidinen des Typs OPC A2 aufweisen. Ein weitere Aufgabe der Erfindung hat darin bestanden, Nahrungsmittelzusatzstoffe zu entwickeln, welche bei oraler Aufnahme sowohl der Hautalterung als auch Entzündungserscheinungen entgegenwirken.

### Beschreibung der Erfindung

Verfahren zur Herstellung pflanzlicher Extrakte, welche Gegenstand der Erfindung sind - bezogen auf den Aktivsubstanzgehalt - wenigstens 15 und vorzugsweise 20 bis 25 Gew.-% an oligomeren Proanthocyanidinen des Typs OPC A2 enthalten. Die Extrakte sind dadurch erhältlich, daß man
(a) Schalen der Früchte von *Litchi sinensis* einer Extraktion mit niederen, gegebenenfalls wässrigen aliphatischen Alkoholen unterwirft,
(b) die Extrakte gegebenenfalls nach Konzentration und/oder Filtration einer chromatographischen Trennung unterzieht,
(c) die bei der Chromatographie anfallende OPC A2-reiche Fraktion einer Flüssig/Flüssig-Extraktion unterwirft und
(d) die resultierende organische Phase abtrennt.

Überraschenderweise wurde gefunden, dass durch Kombination der Prozessschritte "Extraktion", "Chromatographie" und "Flüssig/Flüssig-Extraktion" nunmehr Extrakte zugänglich sind, die die OPC A2 in deutlich höheren Konzentrationen enthalten als Produkte des Stands der Technik und die damit in ihrer MMP-inhibierenden und anti-inflammatorischen Wirkung deutlich leistungsstärker sind.

Ein Gegenstand der Erfindung betrifft ein Verfahren zur Herstellung pflanzliche Extrakte enthaltend - bezogen auf den Aktivsubstanzgehalt - wenigstens 15 und vorzugsweise 20 bis 25 Gew.-% an oligomeren Proanthocyanidinen des Typs OPC A2, bei dem man
(a) Schalen der Früchte von *Litchi sinensis* einer Extraktion mit niederen, gegebenenfalls wässrigen aliphatischen Alkoholen unterwirft,
(b) die Extrakte gegebenenfalls nach Konzentration und/oder Filtration einer chromatographischen Trennung unterzieht,
(c) die bei der Chromatographie anfallende OPC A2-reiche Fraktion einer Flüssig/Flüssig-Extraktion unterwirft und
(d) die resultierende organische Phase abtrennt.

### Extraktion

Die Herstellung der Extrakte kann in an sich bekannter Weise erfolgen, d.h. beispielsweise durch wässrigen, alkoholischen oder wässrig-alkoholischen Auszug der Pflanzen bzw. Pflanzenteile bzw. Schalen der Litchifrüchte. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial wird vorzugsweise von Litchischalen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol sowie deren wässrigen Gemischen. Die Extraktion erfolgt in der Regel bei 20 bis 100 °C, bevorzugt bei 50 bis 70 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte stellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion der Litschischalen liegen bezogen auf OPC A2 im Bereich von 2 bis 3 Gew.-%.

### Chromatographie und Flüssig/Flüssig Extraktion

Die chromatographische Reinigung und die Flüssig/Flüssig-Extraktion können in an sich bekannter Weise durchgeführt werden. Als Säulenmaterial für die Chromatographie haben sich Harze bewährt, die keine funktionellen Gruppen tragen. Die Trennung wird vorzugsweise bei 15 bis 30 °C durchgeführt, wobei als Laufmittel vor allem niedere aliphatische Alkohole mit 1 bis 4 Kohlenstoffatomen, insbesondere Methanol oder Ethanol in Betracht kommen. Für die nachfolgende Extraktion haben sich mit Wasser nicht mischbare Lösemittel bewährt, wie beispielsweise Butanol oder Ethylacetat. Die Extraktion wird dabei vorzugsweise bei Temperaturen von wenigstens 25 °C durchgeführt, wobei sich die obere Grenze aus dem Siedepunkt des Lösemittels ergibt.

### Gewerbliche Anwendbarkeit

Extrakte von *Litchi sinensis* im allgemeinen und die OPC A2-angereicherten neuen Extrakte im besonderen, verfügen gegenüber Produkten des Stands der Technik über eine höhere MMP-Inhibierung und eignen sich, beispielsweise unter dem Oberbegriff "cosmetic inside" zur Herstellung von Nahrungsmittelergänzungsstoffen. In einer besonderen Darreichungsform werden die Extrakte in verkapselter Form, z.B. als Gelatinekapseln, oder in mikroverkapselter Form eingesetzt. Geeignete Mikrokapseln mit Durchmessern in Bereich von 0,0001 bis 5 mm und Verfahren zu deren Herstellung sind beispielsweise den Druckschriften WO 01/01926, WO 01/01927, WO 01/01928, und WO 01/01929 (Primacare) zu entnehmen.

Ein weiterer Gegenstand der vorliegenden Erfindung betrifft des weiteren die Verwendung der neuen OPC-A2 reichen Extrakte zur Herstellung von kosmetischen und/oder pharmazeutischen Zubereitungen, in denen sie in Mengen von 0,1 bis 10, vorzugsweise 0,5 bis 5 und insbesondere 1 bis 2 Gew.-% enthalten sein können.

### Beispiele

### Beispiel 1

In einem 50-1-Reaktor wurden 3 kg zerkleinerte Schalen von *Litchi sinensis* mit einem Gehalt an OPC A2 von 0,4 Gew.-% vorgelegt und mit 30 kg wässrigen Methanol 60 min bei 50 °C extrahiert. Es wurden 23 kg einer Flüssigkeit mit einem Feststoffgehalt von 500 g erhalten, welcher einen OPC A2 Gehalt von 2 Gew.-% aufwies. Nach Aufkonzentrieren des Extraktes auf ein Volumen von 1,5 kg wurde die Flüssigkeit einer chromatographischen Reinigung bei 25 °C auf einer Säule mit einem Belag ohne funktionelle Gruppen und unter Einsatz von Ethanol als Laufmittel unterworfen, bei der 2,5 kg Extrakt mit einem Feststoffanteil von 80 g erhalten wurde. Der Extrakt wurde erneut bis auf ein Volumen von 500 g eingeengt und dann bei 45 °C einer Flüssig/Flüssig-Chromatographie mit wässrigem Butanol unterworfen. Während die intensiv rot gefärbte wässrige Phase nur - bezogen auf den Aktivsubstanzgehalt - Mengen kleiner 1 Gew.-% an OPC A2 aufwies, wurden 50 g einer leicht rötlich gefärbten organischen Phase erhalten, welche - bezogen auf den Aktivsubstanzgehalt - einen Gehalt an OPC A2 von 23 Gew.-% aufwies.

### Vergleichsbeispiel V1

Analog Beispiel 1 wurden in einem 50-1-Reaktor 3 kg zerkleinerte Schalen von *Litchi sinensis* mit einem Gehalt an OPC A2 von 0,4 Gew.-% vorgelegt und mit 30 kg wässrigem Methanol 60 min bei 50 °C extrahiert. Nach Abtrennen des Methanols wurde ein Rückstand von 500 g erhalten, der in 1,5 1 destilliertem Wasser aufgenommen wurde. Anschließend wurde diese Lösung mehrfach mit in Summe 3 1 Ethylacetat extrahiert. Dabei wurde nach Trennung eine leicht rötlich gefärbte organische Phase erhalten, welche - bezogen auf den Aktivsubstanzgehalt - einen Gehalt an OPC A2 von 10 Gew.-% aufwies.

### Wirksamkeit gegen Proteasen

Während einer Inflammation, werden aus den polymorphonuclearen neutrophilen Granulocyten oder Makrophagen Hautproteasen, wie beispielsweise Collagenase freigesetzt. Ein ähnlicher Vorgang spielt sich gerade in der Haut älterer Menschen bei Einfluss von UV-Strahlen ab. Die Proteasen - wegen ihres Gehaltes an zentralen Zinkionen auch als Matrix-Metallo-Proteasen (MMP) bezeichnet - katalysieren wie schon erwähnt die Fragmentierung von Bindegewebsproteinen. Zur Untersuchung der Testsubstanzen auf Collagenaseinhibierung wurde bakterielle Collagenase (Clostridium histolyticum) auf Gelatine als natürlichem Nährboden verwendet, welche mit Fluorochrom (FITC, Calbiochem) markiert war. Die Inkubationszeit betrug 60 min bei 20 °C, die Hydrolyse des Substrates wurde über die Fluoreszenz bei 393 nm (Anregung bei 328 nm) verfolgt. Die Ergebnisse sind in Tabelle 1 zusammengefasst. Angegeben ist die Collagenase-Inhibierung in %.

**Tabelle 1**

| **Collagenase-Inhibierung (Angabe in %-rel.)** | | | | |
|---|---|---|---|---|
| **Bsp.** | **Testprodukt** | **Konzentration % (w/v)** | | |
| | | 0,001 | 0,005 | 0,01 |
| 2 | Produkt gemäß Beispiel 1 | 18 | 46 | 67 |
| V2 | Produkt gemäß Beispiel V1 | 11 | 27 | 34 |

Die Ergebnisse zeigen, dass die erfindungsgemäßen Testsubstanzen in Abhängigkeit der Konzentration über eine signifikante Inhibierungswirkung verfügen.

## Patentansprüche

1. Verfahren zur Herstellung pflanzlicher Extrakte mit wenigstens 15 Gew.-% an oligomeren Proanthocyanidinen des Typs OPC A2, bei dem man
(a) Schalen der Früchte von *Litchi sinensis* einer Extraktion mit niederen, gegebenenfalls wässrigen aliphatischen Alkoholen unterwirft,
(b) die Extrakte gegebenenfalls nach Konzentration und/oder Filtration einer chromatographischen Trennung unterzieht,
(c) die bei der Chromatographie anfallende OPC A2-reiche Fraktion einer Flüssig/Flüssig-Extraktion unterwirft und
(d) die resultierende organische Phase abtrennt.

2. Verfahren nach Anspruch 1, **dadurch gekennzeichnet, dass** man gegebenenfalls wässrigen Methanol oder Ethanol einsetzt.

3. Verfahren nach den Ansprüchen 1 oder 2, **dadurch gekennzeichnet, dass** man die Extraktion bei Temperaturen im Bereich von 30 bis 70 °C durchführt.

4. Verfahren nach mindestens einem der Ansprüche 1 bis 3, **dadurch gekennzeichnet, dass** man die chromatographische Trennung auf einer Säule durchführt, deren Belag keine funktionellen Gruppen aufweist.

5. Verfahren nach mindestens einem der Ansprüche 1 bis 4, **dadurch gekennzeichnet, dass** man die chromatographische Trennung mit Laufmitteln durchführt, die ausgewählt sind aus der Gruppe der aliphatischen Alkohole mit 1 bis 4 Kohlenstoffatomen.

6. Verfahren nach mindestens einem der Ansprüche 1 bis 5, **dadurch gekennzeichnet, dass** man die Flüssig/Flüssig-Extraktion unter Verwendung von nicht mit Wasser mischbaren Lösungsmitteln durchführt.

## Claims

1. A process for the production of botanic extracts containing at least 15% by weight of oligomeric proanthocyanidins of the OPC A2 type, **characterized in that** it comprises the steps of
(a) subjecting shells of the fruit of *Litchi sinensis* to extraction with lower, optionally aqueous aliphatic alcohols,
(b) subjecting the extracts to chromatographic separation, optionally after concentration and/or filtration,
(c) subjecting the OPC A2-rich fraction obtained in the chromatography step to a liquid/liquid extraction and
(d) removing the resulting organic phase.

2. A process as claimed in claim 1, **characterized in that** optionally aqueous methanol or ethanol is used.

3. A process as claimed in claim 1 or 2, **characterized in that** the extraction is carried out at temperatures of 30 to 70°C.

4. A process as claimed in at least one of claims 1 to 3, **characterized in that** the chromatographic separation is carried out in a column of which the coating has no functional groups.

5. A process as claimed in at least one of claims 1 to 4, **characterized in that** the chromatographic separation is carried out with mobile solvents selected from the group of aliphatic alcohols containing 1 to 4 carbon atoms.

6. A process as claimed in at least one of claims 1 to 5, **characterized in that** the liquid/liquid extraction is carried out using water-immiscible solvents.

## Revendications

1. Procédé de préparation d'extraits végétaux renfermant au moins 15 % en poids de proanthocyanidines oligomériques du type OPC A2, selon lequel
(a) on soumet les écorces des fruits de *Litchi sinensis* à une extraction avec des alcools aliphatiques inférieurs, éventuellement aqueux,
(b) on soumet les extraits éventuellement après concentration et/ou filtration à une séparation chromatographique,
(c) on soumet la fraction riche en OPC A2 obtenue par la chromatographie à une extraction liquide/liquide, et
(d) on sépare la phase organique obtenue.

2. Procédé selon la revendication 1,
**caractérisé en ce qu'**
on utilise du méthanol ou de l'éthanol éventuellement aqueux.

3. Procédé selon les revendications 1 ou 2,
**caractérisé en ce qu'**
on effectue l'extraction à des températures dans la gamme de 30 à 70 °C.

4. Procédé selon au moins une des revendications 1 à 3,
**caractérisé en ce qu'**
on effectue la séparation chromatographique sur une colonne dont la phase stationnaire ne présente pas de groupes fonctionnels.

5. Procédé selon au moins une des revendications 1 à 4,
**caractérisé en ce qu'**
on effectue la séparation chromatographique avec des phases mobiles choisies dans le groupe des alcools aliphatiques ayant de 1 à 4 atomes de carbone.

6. Procédé selon au moins une des revendications 1 à 5,
**caractérisé en ce qu'**
on effectue l'extraction liquide/liquide en utilisant des solvants non miscibles à l'eau.
